# EUROPÄISCHE PATENTANMELDUNG

(11) **EP 1 574 859 A1**
(43) Veröffentlichungstag der Anmeldung: **14.09.2005**
(21) Anmeldenummer: 05004887.5
(22) Anmeldetag: 05.03.2005
(51) Int. Cl.: G01N 33/58, G01N 33/543

(54) **Verfahren zur Charakterisierung von künstlichen Oberflächen**

(30) Priorität: 10.03.2004 DE 102004013052
(71) Anmelder: Fraunhofer-Gesellschaft zur Förderung der angewandten Forschung e.V., 80686 München (DE)
(72) Erfinder: Schiestel, Thomas, Dr., 70565 Stuttgart (DE); Brunner, Herwig, Prof. Dr., 70569 Stuttgart (DE); Rupp, Steffen, Dr., 70195 Stuttgart (DE)
(74) Vertreter: Schrell, Andreas

(57) **Zusammenfassung**

Die Erfindung betrifft ein Verfahren zur Charakterisierung von Nanodomänen aufweisenden Oberflächen, wobei Biosensoren, die an Nanopartikeln immobilisierte Affinitätsmoleküle aufweisen, mit einer Nanodomänen aufweisenden Oberfläche inkubiert werden und die Bindung der Biosensoren an eine Nanodomäne der Oberfläche mittels einer Detektionseinrichtung charakterisiert wird.

## Beschreibung

Die vorliegende Erfindung betrifft Verfahren zur Charakterisierung von Oberflächen, die Nanodomänen aufweisen.

Beim Einsatz technischer Oberflächen in biologischer Umgebung (Implantate, Katheder, Dialysemembranen) spielen verschieden Faktoren für die Biokompatibilität eine Rolle. Neben der chemischen Zusammensetzung der Werkstoffe und deren Auswirkungen auf die biologische Umgebung (zum Beispiel Toxizität) sowie deren makroskopisch-mechanischer Eigenschaften (zum Beispiel Härte) ist die Mikro- und Nanostruktur der Oberfläche entscheidend für die Biokompatibilität, insbesondere was die Aktivierung von Gerinnungsfaktoren oder Zelladhäsion betrifft.

Dialysemembranen für die Nierenwäsche werden beispielsweise aus binären oder ternären Polymermischungen hergestellt. Es wird vermutet, dass es beim Spinprozess zu einer zumindest teilweisen Entmischung kommt, sodass letztendlich Nanodomänen unterschiedlicher Polymerer nebeneinander vorliegen, deren Größe und Zusammensetzung wiederum die Aktivierung von Gerinnungsfaktoren entscheidend beeinflusst. Nur wenn sich diese Nanodomänen gut charakterisieren lassen, ist der Herstellungsprozess optimierbar.

Der vorliegenden Erfindung liegt daher das technische Problem zugrunde, Verfahren zur Verfügung zu stellen, die es ermöglichen, Nanodomänen aufweisende Oberflächen unterschiedlichster Werkstoffe oder Werkstücke zu charakterisieren, wobei diese Charakterisierung eine Identifikation und/oder den Nachweis eines oder mehrerer bekannter Materialien in oder an der Oberfläche des Werkstoffs oder Werkstücks gewährleisen sollte.

Die vorliegende Erfindung löst dieses technisches Problem durch die Bereitstellung eines Verfahrens zur Charakterisierung von Nanodomänen aufweisenden Oberflächen, wobei Biosensoren, die an Nanopartikeln immobilisierte Affinitätsmoleküle aufweisen, mit einer die Nanodomänen aufweisenden Oberfläche inkubiert und deren Bindung an die Oberfläche mittels einer Detektionseinrichtung charakterisiert, insbesondere nachgewiesen und analysiert wird. Die Charakterisierung kann eine qualitative und/oder quantitative Charakterisierung sein.

Im Zusammenhang mit der vorliegenden Erfindung ist unter einer Nanodomäne ein räumlich abgegrenzter Bereich zu verstehen, innerhalb dessen ein homogenes Material oder/und homogene Materialeigenschaften vorliegen und der eine Größe von 1 bis 1000 nm, vorzugsweise 1 bis 100 nm, aufweist.

Die vorliegende Erfindung setzt zur Charakterisierung von Nanodomänen aufweisenden Oberflächen daher Biosensoren ein, welche an Nanopartikeln immobilisierte Affinitätsmoleküle umfassen, beziehungsweise aus solchen Nanopartikel-gebundenen Affinitätsmolekülen bestehen. Ein Biosensor stellt eine Kombination eines bestimmten Nanopartikels mit einem bestimmten Affinitätsmolekül dar. In einer bevorzugten Ausführungsform bestehen die Biosensoren aus Nanopartikeln und daran immobilisierten Affinitätsmolekülen, enthalten also keine weiteren Bestandteile. Es kann auch vorgesehen sein, dass die Nanopartikel über Linker oder Spacer mit den Affinitätsmolekülen verbunden sind. Die Immobilisierung der Affinitätsmoleküle an die Nanopartikel kann kovalent oder nicht-kovalent sein, wobei auch vorgesehen sein kann, dass die Bindung in Abhängigkeit von den Reaktionsbedingungen gelöst oder eingegangen wird, das heißt eine reversible Bindung ist. Die an den Nanopartikeln immobilisierten Affinitätsmoleküle lassen sich über evolutionäre und/oder kombinatorische Verfahren gezielt so herstellen, dass sie Materialien, insbesondere an und/oder in Oberflächen, mit hoher Selektivität und Sensitivität binden und erkennen können. Nach Inkubation der zu charakterisierenden Oberflächen mit den erfindungsgemäß eingesetzten Biosensoren, welche bestimmte Nanodomänen selektiv mit hoher Sensitivität erkennen, kann anschließend mittels einer Detektionseinrichtung die Verteilung von Nanodomänen, zum Beispiel mittels eines Elektronenmikroskops eindeutig und leicht charakterisiert beziehungsweise identifiziert werden. Die erfindungsgemäß vorgesehene Verfahrensweise ermöglicht die Erfassung unterschiedlichster Nanodomänen auf Oberflächen in einfacher und zerstörungsfreier Art und Weise. Die erfindungsgemäße Vorgehensweise eignet sich daher besonders gut zur Materialprüfung, um frühzeitig aufgetretene Produktionsmängel rechtzeitig erkennen und folglich vermeiden zu können.

Im Zusammenhang mit der vorliegenden Erfindung werden unter Affinitätsmolekülen solche Moleküle oder Molekülverbände verstanden, die in der Lage sind, Nanodomänen aufweisende Oberflächen, insbesondere solche aus weichen, nicht-elektronendichten Materialien eindeutig zu charakterisieren, insbesondere Nanodomänen mit hoher Sensitivität und Selektivität zu erkennen. Erfindungsgemäß werden vorzugsweise als Affinitätsmoleküle Oligonucleotide, Polynucleotide, Nucleotidderivate, natürliche oder synthetische Antikörper oder deren Fragmente oder Derivate, Aptamere, das heißt Oligonucleotidliganden, Lektine, Peptide, Oligopeptide, Polypeptide, Aminosäurederivate, Glycoproteine, Cofaktoren, Vitamine, Enzyme, Bakterien, Viren, Bestandteile von Bakterien oder Viren, Viroide, Prionen, Zellbestandteile, Proteoglycane, Lipide, Kohlenhydrate, Oligosaccharide, Polysaccaride, Glycosaccharide und/oder heterozyklische Verbindungen eingesetzt. Die Affinitätsmoleküle können auch chemische oder biologische Abwandlungen oder Derivate der vorgenannten Moleküle oder Molekülverbände sein.

Die Erfindung sieht in bevorzugter Ausführung vor, dass die Affinitätsmoleküle über evolutionäre Verfahren wie sie in Ellington A. D. and Szostak J. W. (1990) Nature 346, 818-822, Joyce, G. F. (1989) Gene 82, 83-87, Tuerk, C. and Gold, L. (1990) Science 249, 505-510 beschrieben sind, zum Beispiel SELEX oder Phage Display oder mittels kombinatorischer Methoden hergestellt werden können, und zwar in einer Art und Weise, dass sie in der Lage sind, Oberflächen mit hoher Selektivität und Sensitivität für eine bestimmte Nanodomäne zu erkennen. Selbstverständlich können auch in bereits vorhandenen kombinatorischen Bibliotheken enthaltene Affinitätsmoleküle verwendet werden.

Im Zusammenhang mit der vorliegenden Erfindung werden unter Nanodomänen aufweisenden Oberflächen solche Oberflächen verstanden, die in einem Größenbereich von 1 bis 1000 nm, vorzugsweise 1 bis 500 nm, insbesondere 1 bis 100 nm, Strukturen, zum Beispiel Nanodomänen, aufweisen oder solche Domänen aufweisen können. Vorzugsweise sind die Oberflächen glatt oder rauh. Die erfindungsgemäße Vorgehensweise erlaubt das Auffinden derartiger Nanodomänen, das Auffinden von Abweichungen von bekannten oder vorgegebenen Nanodomänen sowie auch den Nachweis, dass bestimmte Nanodomänen nicht oder gar keine Nanodomänen vorhanden sind.

Die Erfindung setzt in vorteilhafter Weise metallische oder polymere Nanopartikel ein, beispielsweise Goldnanopartikel, oder Nanopartikel aus polymerem Material. Es kann auch vorgesehen sein, molekular geprägte Nanopartikel einzusetzen, insbesondere solche, wie sie in der WO 02/02647 A1 beschrieben sind. Bei Verwendung der in der WO 02/02647 A1 beschriebenen molekülspezifische Erkennungsstellen aufweisenden Nanopartikeln ist es erfindungsgemäß möglich, auf die Affinitätsmoleküle zu verzichten, da die dort beschriebenen Nanopartikel bereits in der Lage sind, selektiv und sensitiv Nanostrukturen zu erkennen. Die Nanopartikel weisen bevorzugt eine Größe von 1 bis 1000 nm, insbesondere 1 bis 500 nm und vorzugsweise von 5 bis 100 nm auf. In bevorzugter Ausführung sind die Nanopartikel kleiner als die zu charakterisierenden Nanodomänen.

Die Erfindung ermöglicht es, Oberflächen beliebiger Objekte zu charakterisieren, insbesondere die Oberfläche eines Implantats, Katheders, einer Dialysemembran, einer Sensoroberfläche, Stents, einer Filtermembran, Hohlfasermoduls, Metalls, Halbmetalls, wie Silizium, Metalloxids, Glases, Papiers, eines Kunststoffs wie Polyester, einer mikroporösen Keramik, von verflochtenen oder verfilzten Fasern, eines Filters oder einer Folie. Die Erfindung betrifft also die Charakterisierung von Materialien oder Materialeigenschaften, die an Oberflächen eines beliebigen Objektes insbesondere Werkstückes angeordnet und/oder charakterisierbar sind. Die Erfindung stellt demgemäß also ein Materialprüfungsverfahren zur Verfügung, dass sich das vorstehende Verfahren zur Charakterisierung von Nanodomänen aufweisenden Oberflächen zu Nutze macht. Derartige Oberflächen können die üblichen Oberflächen eines Werkstückes sein, es können aber auch Oberflächen sein, die zum Beispiel erst nach Bearbeitung des Werkstückes entstehen oder freigelegt oder sonst wie zugänglich gemacht werden. Die Erfindung betrifft daher auch die Charakterisierung von inneren Oberflächen eines Werkstückes, die beispielsweise erst nach Ausschneiden oder Aufbrechen eines Werkstückes einer Charakterisierung durch das erfindungsgemäße Verfahren zugänglich gemacht werden.

Erfindungsgemäß kann vorgesehen sein, dass für die erfindungsgemäße Verfahrensweise Nanopartikel ein und derselben Größe und/oder Art zusammen mit Affinitätsmolekülen ein und derselben Art eingesetzt werden. Erfindungsgemäß kann jedoch auch vorgesehen sein, dass hinsichtlich Größe und/oder Art unterschiedliche Nanopartikel im Rahmen eines erfindungsgemäßen Verfahrens mit ein und derselben Art von Affinitätsmolekülen eingesetzt wird oder dass unterschiedliche Arten und/oder Größen von Affinitätsmolekülen mit ein und derselben Art an Nanopartikeln oder hinsichtlich Größe und/oder Art unterschiedliche Nanopartikel mit unterschiedlichen Affinitätsmolekülen zusammen eingesetzt werden.

Demgemäß betrifft die Erfindung den Einsatz eines bestimmten Biosensors, dass heisst einer bestimmten Kombination aus einem bestimmten Nanopartikel und einem bestimmten Affinitätsmolekül. Die Erfindung betrifft aber auch den Einsatz eines Cocktails oder einer Mischung verschiedener Biosensoren, wie sie vorstehend beschrieben aufgebaut sein können, zum Beispiel den Einsatz von 2 oder mehr verschiedenen Biosensoren gleichzeitig oder sequentiell zum Nachweis von Nanodomänen in oder an Oberflächen.

Erfindungsgemäß kann auch vorgesehen sein, die Nanopartikel mit weiteren Komponenten, zum Beispiel Farb- oder Isotopenmarkierungen, zu versehen.

In bevorzugter Weise werden die Biosensoren in Suspension, zum Beispiel mit einem physiologischen Suspensionsmittel, mit den zu charakterisierenden Oberflächen inkubiert.

Weitere vorteilhafte Ausgestaltungen ergeben sich aus den Unteransprüchen.

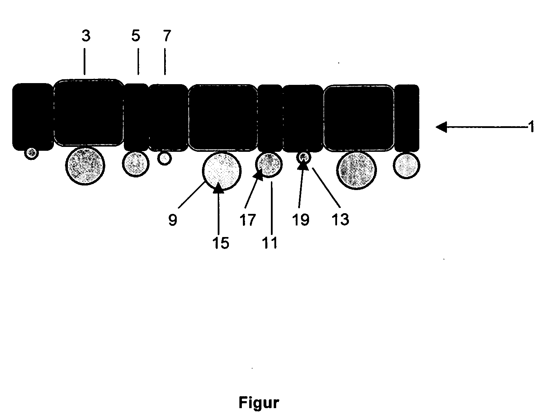

Die anliegende Figur erläutert beispielhaft die vorliegende Erfindung. Der Figur kann eine im wesentlichen glatte Oberfläche 1 mit unterschiedlichen Polymernanodomänen 3, 5 und 7 entnommen werden, die von einer Mischung dreier unterschiedlicher Biosensoren 9, 11 und 13 erkannt werden. Die Biosensoren unterschieden sich einerseits hinsichtlich der Größe des eingesetzten Goldnanopartikels 15, 17 und 19, andererseits aber auch hinsichtlich der Art des auf diesem Goldnanopartikel immobilisierten Affinitätsmoleküls voneinander.

## Patentansprüche

1. Verfahren zur Charakterisierung von Nanodomänen aufweisenden Oberflächen, wobei Biosensoren, die an Nanopartikeln immobilisierte Affinitätsmoleküle aufweisen, mit einer Nanodomänen aufweisenden Oberfläche inkubiert werden und die Bindung der Biosensoren an eine Nanodomäne der Oberfläche mittels einer Detektionseinrichtung charakterisiert wird.

2. Verfahren nach Anspruch 1, wobei das Affinitätsmolekül ein Oligonucleotid, Polynucleotid, ein natürlicher oder synthetischer Antikörper oder Fragment davon, ein Aptamer, ein Lektin, ein Peptid, ein Oligopeptid, ein Polypeptid, ein Aminosäurederivat, ein Nucleotid, ein Glycoprotein, ein Cofaktor, ein Vitamin, ein Enzym, ein Bakterium, ein Virus, ein Viroid, ein Prion, Zellbestandteil, ein Proteoglucan, ein Lipid, ein Kohlenhydrat, ein Oligosaccharid, ein Polysaccharid, ein Glycosaccharid oder eine heterozyklische Verbindung oder eine Abwandlung oder Derivat davon ist.

3. Verfahren nach einem der vorhergehenden Ansprüche, wobei die Oberfläche die Oberfläche eines Implantats, Katheders, einer Dialysemembran, einer Sensoroberfläche, Stents, einer Filtermembran, Hohlfasermoduls, Metalls, Halbmetalls, wie Silizium, Metalloxids, Glases, Papiers, eines Kunststoffs wie Polyester, einer mikroporösen Keramik, verflochtener oder verfilzter Fasern, eines Filters oder einer Folie ist.

4. Verfahren nach einem der vorhergehenden Ansprüche, wobei die Nanopartikel metallische oder polymere Nanopartikel sind, insbesondere Goldnanopartikel.

5. Verfahren nach einem der vorhergehenden Ansprüche, wobei das Affinitätsmolekül aus einer kombinatorischen Bibliothek stammt oder mittels eines kombinatorischen und/oder evolutionären Verfahrens hergestellt wurde.
